# EUROPEAN PATENT APPLICATION

(11) **EP 4 579 004 A1**
(43) Date of publication of application: **02.07.2025**
(21) Application number: 24210031.1
(22) Date of filing: 31.10.2024
(51) Int. Cl.: C30B 23/02, C30B 29/36

(54) **SIC INGOT AND METHOD FOR MANUFACTURING SIC SUBSTRATE**

(30) Priority: 28.12.2023 JP 2023223461; 28.12.2023 JP 2023223189; 28.12.2023 JP 2023223451; 25.10.2024 JP 2024188125; 25.10.2024 JP 2024188186; 25.10.2024 JP 2024188277
(71) Applicant: Resonac Corporation, Tokyo 105-7325 (JP)
(72) Inventor: Kamei, Koji, Tokyo, 105-7325 (JP); Nagahata, Yukio, Tokyo, 105-7325 (JP)
(74) Representative: Strehl & Partner mbB

(57) **Abstract**

This SiC ingot is formed of a SiC single crystal grown from a first end inclined by an offset angle from a (0001) plane toward a second end, the SiC ingot includes a step-flow growth region and a facet, and in a cut surface that passes through a center and is in a <11-20> direction, an angle between an inner boundary between the facet and the step-flow growth region and a crystal growth direction is 56° or less.

## Description

### BACKGROUND OF THE INVENTION

### Field of the Invention

The present invention relates to a SiC ingot and a method for manufacturing a SiC substrate.

The present application claims priority on Japanese Patent Application No. 2023-223189 filed on December 28, 2023, Japanese Patent Application No. 2023-223451 filed on December 28, 2023, Japanese Patent Application No. 2023-223461 filed on December 28, 2023, Japanese Patent Application No. 2024-188125 filed on October 25, 2024, Japanese Patent Application No. 2024-188186 filed on October 25, 2024, Japanese Patent Application No. 2024-188277 filed on October 25, 2024, the content of which is incorporated herein by reference.

### Description of Related Art

Silicon carbide (SiC) has a dielectric breakdown field one order of magnitude larger than silicon (Si) and a band gap three times larger than silicon (Si). In addition, silicon carbide (SiC) has properties such as a thermal conductivity about three times higher than silicon (Si). For this reason, silicon carbide (SiC) is expected to be applied to power devices, high frequency devices, high temperature operating devices, and the like. For this reason, in recent years, SiC epitaxial wafers have come to be used for the above-described semiconductor devices.

A SiC epitaxial wafer is obtained by stacking a SiC epitaxial layer on the surface of a SiC substrate. The SiC substrate is cut out from a SiC ingot. A SiC ingot is obtained by performing crystal growth of a SiC single crystal on a seed crystal. When the crystal growth of the SiC single crystal is performed on the seed crystal, a facet and a step-flow growth region are formed.

Patent Document 1 describes a method for manufacturing a SiC single crystal. In addition, Patent Document 1 describes that, from an initial stage to a middle stage of the growth, if the temperature of a facet region is made lower than that of a non-facet region, or the raw material gas concentration of the facet region is made higher than that of the non-facet region, then the growth of the facet region and its neighboring regions is promoted and the area of the facet region is reduced.

### [Patent Documents]

[Patent Document 1] Japanese Patent No. 6050053

### SUMMARY OF THE INVENTION

In recent years, laser processing of a SiC ingot has been carried out. For example, cracks are created in a SiC ingot with a laser, and a SiC substrate is cut out from the SiC ingot. The optimum value of a laser output when cutting out the SiC substrate using the laser varies depending on the resistance value of the SiC single crystal. If the laser output is low, sufficient cracks are not generated. In addition, if the laser output is too high, the SiC single crystal will be damaged.

The facet of the SiC ingot has a lower resistance value than the step-flow growth region obtained through step-flow growth. Therefore, in a case in which a SiC substrate is cut out from the SiC ingot, it is necessary to change the laser output at a boundary between the facet and the step-flow growth region. The greater the number of times that the laser output is changed in the process of cutting out the SiC substrate, the lower the productivity.

The present invention has been made in consideration of the above-described problems, and an object of the present invention is to provide a SiC ingot which is easily laser-processed and a method for manufacturing a SiC substrate using the SiC ingot.

The present inventors have focused on the shape of a facet of a SiC ingot and conducted extensive research in order to improve productivity by reducing the number of times that the laser output is changed when cutting out a SiC substrate from a SiC ingot by laser processing. As a result, they found that it would be sufficient to produce a SiC ingot having a facet controlled to a specific shape, and thus they made the present invention. The present invention provides the following solutions.
[1] A SiC ingot according to a first aspect is formed of a SiC single crystal grown from a first end inclined by an offset angle from a (0001) plane toward a second end. The SiC ingot includes a step-flow growth region and a facet. In a cut surface that passes through a center and is in a <11-20> direction, an angle between an inner boundary between the facet and the step-flow growth region and a crystal growth direction is 56° or less.
[2] In the SiC ingot according to the above aspect [1], in the inner boundary extending from the first end toward the second end, an angle inclined in a [-1-120] direction with respect to a thickness direction from the first end toward the second end may be more than 0° and 56° or less.
[3] In the SiC ingot according to the above aspect [1] or [2], a diameter may be 149 mm or more.
[4] In the SiC ingot according to the above aspect [1] or [2], a diameter may be 199 mm or more.
[5] In the SiC ingot according to any one of the above aspects [1] to [4], a maximum thickness between the first end and the second end in a direction perpendicular to the first end may be 10 mm or more.
[6] In the SiC ingot according to any one of the above aspects [1] to [4], a maximum thickness between the first end and the second end in a direction perpendicular to the first end may be 20 mm or more.
[7] In the SiC ingot according to any one of the above aspects [1] to [4], a maximum thickness between the first end and the second end in a direction perpendicular to the first end may be 30 mm or more.
[8] In the SiC ingot according to any one of the above aspects [1] to [4], a maximum thickness between the first end and the second end in a direction perpendicular to the first end may be 40 mm or more.
[9] The SiC ingot according to any one of the above aspects [1] to [4], a maximum thickness between the first end and the second end in a direction perpendicular to the first end may be 50 mm or more.
[10] A method for manufacturing a SiC substrate according to a second aspect includes:
   a step of manufacturing the SiC ingot according to any one of the above aspects [1] to [9];
   a step of processing the SiC ingot into a cylindrical shape; and
   a step of slicing the SiC ingot that has been processed into a cylindrical shape.
[11] A method for manufacturing a SiC substrate according to a third aspect includes:
   a step of preparing the SiC ingot according to any one of the above aspects [1] to [9]; and
   a step of slicing the SiC ingot.
[12] A method for evaluating a SiC ingot according to a fourth aspect includes:
   a step of manufacturing a SiC ingot;
   a step of processing the SiC ingot into a cylindrical shape;
   a step of slicing the SiC ingot that has been processed into a cylindrical shape to obtain a plurality of SiC substrates for evaluation;
   a step of measuring a position of an inner boundary between a facet and a step-flow growth region in each SiC substrate for evaluation;
   a step of calculating an angle between the inner boundary between the facet and the step-flow growth region in a cut surface that passes through a center of the SiC ingot and is in a <11-20> direction and a crystal growth direction on the basis of a relationship between a position in the SiC ingot from which each of the SiC substrates for evaluation is cut out and a position of the inner boundary of each of the SiC substrates for evaluation; and
   an evaluation step of evaluating whether the SiC ingot is produced under conditions suitable for manufacturing a SiC ingot to be sliced by laser processing on the basis of the angle between the inner boundary and the crystal growth direction.

The SiC ingot according to the above aspect is formed of a SiC single crystal grown from a first end inclined by an offset angle from a (0001) plane toward a second end, and in a cut surface that passes through a center and is in a <11-20> direction, an angle between an inner boundary between a facet and a step-flow growth region and a crystal growth direction is 56° or less. For this reason, in the SiC ingot according to the above aspect that is processed into a cylindrical shape, it is possible to reduce the number of times that the laser output is changed when cutting out the SiC substrate by laser processing, and it is possible to easily and efficiently cut out the SiC substrate.

### BRIEF DESCRIPTION OF THE DRAWINGS

FIG. 1 is a cross-sectional view of a SiC ingot according to the present embodiment.
FIG. 2 is a plan view of the SiC ingot according to the present embodiment.
FIG. 3 is a cross-sectional view showing an example of a manufacturing apparatus used when manufacturing the SiC ingot according to the present embodiment.
FIG. 4 is a cross-sectional view showing another example of the manufacturing apparatus used when manufacturing the SiC ingot according to the present embodiment.

### DETAILED DESCRIPTION OF THE INVENTION

Hereinafter, a SiC ingot and a method for manufacturing a SiC substrate according to the present embodiment will be described in detail with reference to the drawings as appropriate. In the drawings which will be used in the following description, featured portions may be enlarged for convenience in order to make the features of the present embodiment easy to understand, and the dimensional ratios of constituent elements may be different from the actual ones. The materials, dimensions, and the like which will be exemplified in the following description are examples, and the present invention is not limited thereto and can be carried out with appropriate modifications without changing the features of the present invention.

In the present specification, an individual orientation is indicated by [ ], a collective orientation is indicated by < >, an individual plane is indicated by ( ), and a collective plane is indicated by { }. For negative indices, a "-" (bar) is customarily placed above the number in crystallography, but in the present specification, a negative sign is placed before the number.

First, directions are defined as follows. A crystal growth direction of a SiC ingot 10 is defined as a Z direction. The Z direction is a height direction of the SiC ingot 10 in an approximately cylindrical shape. One direction in a plane orthogonal to the Z direction is defined as an X direction. The X direction is, for example, a <11-20> direction. For example, a +X direction is defined as a [11-20] direction, and a -X direction is defined as a [-1-120] direction. In addition, in a plane orthogonal to the Z direction, a direction orthogonal to the X direction is defined as a Y direction. The Y direction is, for example, a <1-100> direction.

"SiC ingot"

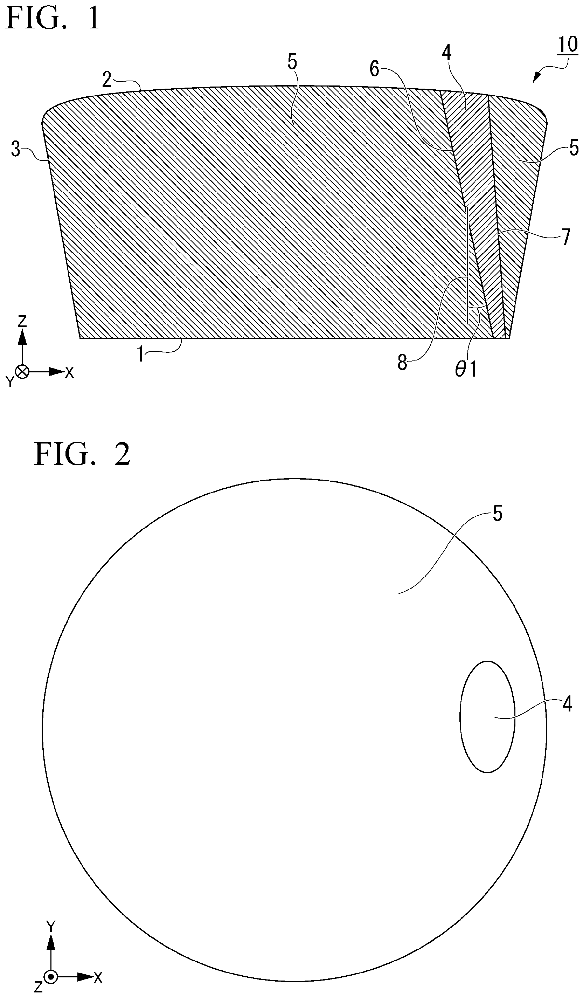

FIG. 1 is a cross-sectional view of the SiC ingot 10 according to the present embodiment. FIG. 2 is a plan view of the SiC ingot 10 according to the present embodiment shown in FIG. 1 in the Z direction.

The SiC ingot 10 has an approximately cylindrical shape and is a single crystal of SiC obtained by performing the crystal growth from a first end 1 toward a second end 2. The SiC ingot 10 may be one that has been processed into a cylindrical shape or one that has not yet been processed into a cylindrical shape. The second end 2 is a terminal in the crystal growth direction. The first end 1 and the second end 2 are connected to each other by a side wall 3. The SiC ingot 10 may expand in diameter from the first end 1 toward the second end 2, or may be cylindrical with a constant diameter.

The first end 1 is a plane inclined by an offset angle from a (0001) plane (a Si plane). The second end 2 is a plane inclined by an offset angle from a (000-1) plane (a C plane). The second end 2 faces the first end 1. The first end 1 and the second end 2 may have an offset angle of, for example, 0.1 degrees or more and 8 degrees or less in the <11-20> direction, and may have no offset angle in the <1-100> direction. The offset angle of the SiC ingot is not limited to this example.

The diameter D of the SiC ingot 10 is, for example, 145 mm or more, preferably 149 mm or more, more preferably 150 mm or more, and further preferably 151 mm or more, and may be 199 mm or more. The diameter D of the SiC ingot 10 is, for example, 305 mm or less. The diameter D of the SiC ingot 10 may be, for example, 230 mm or less, and is preferably 220 mm or less, more preferably 205 mm or less, and further preferably 201 mm or less.

The diameter D of the SiC ingot 10 is the minimum diameter of the SiC ingot 10 and corresponds to the minimum value of the diameters of the SiC substrates that can be acquired from the SiC ingot 10. As shown in FIG. 1, for example, in a case in which the SiC ingot 10 has a shape that expands in diameter from the first end 1 toward the second end 2, the diameter of the first end 1 corresponds to the diameter D of the SiC ingot 10. For example, in a case in which the SiC ingot 10 is cylindrical with a constant diameter, the diameter of any cross section obtained by cutting the SiC ingot 10 along a surface orthogonal to the Z direction corresponds to the diameter D of the SiC ingot 10. The SiC ingot 10 may be, for example, one from which a 6-inch substrate can be acquired, or one from which an 8-inch substrate can be acquired.

The thickness of the SiC ingot 10 is the maximum thickness between the first end 1 and the second end 2 in a direction perpendicular to the first end 1. The thickness of the SiC ingot 10 is, for example, 10 mm or more, preferably 20 mm or more, more preferably 30 mm or more, even more preferably 40 mm or more, further preferably 50 mm or more, and particularly preferably 60 mm or more. The thickness of the SiC ingot 10 may be 100 mm or more. The thicker the SiC ingot 10, the more SiC substrates can be acquired, and thus a thick SiC ingot 10 is preferable. In addition, the thickness of the SiC ingot 10 is, for example, 300 mm or less.

The SiC ingot 10 includes a facet 4 and a step-flow growth region 5.

The SiC ingot 10 includes a SiC seed crystal and a crystal growth portion obtained by performing crystal growth on the SiC seed crystal. As the SiC seed crystal, one having an offset angle with respect to the {0001} plane is used. This is because the occurrence of crystals of heterogeneous polymorphs can be suppressed by performing step-flow growth of a SiC single crystal on the SiC seed crystal. The offset angle of the SiC seed crystal with respect to the {0001 } plane is, for example, 3.5° or more and 4.5° or less, and is preferably 4°.

Even in a case in which the step-flow growth of the SiC single crystal is performed on the SiC seed crystal, during the growth of the SiC single crystal, a part of a crystal growth surface becomes parallel to the (0001) plane, and a surface parallel to the (0001) plane is exposed on the crystal growth surface. On the surface parallel to the (0001) plane, crystals grow perpendicular to the (0001) plane, and thus the step-flow growth is not performed. The facet 4 of the SiC ingot 10 is a region in which the crystals have grown perpendicular to the (0001) plane. As shown in FIG. 2, the facet 4 is an approximately circular region when seen in the Z direction. The step-flow growth region 5 is a region in which the SiC single crystal has grown on the SiC seed crystal through the step-flow growth. As shown in FIG. 2, the step-flow growth region 5 is formed to surround the facet 4. The step-flow growth region 5 has an offset angle with respect to the {0001} plane. The offset angle of the step-flow growth region 5 with respect to the {0001} plane is, for example, 3.5° or more and 4.5° or less, and is preferably 4°.

The facet 4 and the step-flow growth region 5 have different colors when the SiC ingot 10 is seen in a plan view in the Z direction (the crystal growth direction). This is because the facet 4 and the step-flow growth region 5 have different patterns in crystal growth. The facet 4 is visually observed as a region darker in color than the step-flow growth region 5. Therefore, the boundary between the facet 4 and the step-flow growth region 5 can be visually checked.

When the SiC ingot 10 is seen in a plan view in the Z direction, the boundary between the facet 4 and the step-flow growth region 5 can be determined visually, but it may also be determined by the following procedure.

First, an image of the SiC ingot 10 in a plan view in the Z direction is acquired. For example, the image is acquired using a scanner. As the scanner, for example, a flatbed scanner manufactured by Canon Inc. can be used. The image may be acquired using a digital camera.

Next, the acquired image is converted into an HLS color space consisting of hue, luminance, and saturation, and the luminance is calculated. Then, in the image converted into a luminance distribution, a circle of pixels with a radius X is drawn with an arbitrary pixel as the center of the circle. In a case in which there are pixels within this circle with a luminance difference from the central pixel of Y or more, the pixel at the center of the circle becomes a pixel that is a facet candidate. In a case in which there are no pixels within this circle with a luminance difference from the central pixel of Y or more, the pixel at the center of the circle becomes a pixel that is not a facet candidate. A similar treatment is then performed on all pixels of the image, and the pixels are classified as either pixels that are facet candidates or pixels that are not facet candidates. Then, the boundary between the pixels that are facet candidates and the pixels that are not facet candidates is detected, and the inside of this region can be considered as a facet. Among the pixels that are facet candidates, pixels that are isolated from other pixels that are facet candidates can be determined not to be facet candidates. The radius X of the circle and the luminance difference Y are set according to the size and number of pixels of the image. These settings involve setting values that do not cause a large discrepancy between the visual observation result and the determination result. For example, in a case in which a 640 pixel × 480 pixel image including a 150 mm-diameter wafer acquired from the SiC ingot is used, the radius X is set to 9 pixels and the luminance difference Y is set to 4 W·sr⁻¹·m⁻².

Even in an XZ cut surface of the SiC ingot 10, the boundary between the facet 4 and the step-flow growth region 5 can be visually determined. Hereinafter, in the boundary between the facet 4 and the step-flow growth region 5, a boundary located on a side of the center of the SiC ingot 10 in the XZ cut surface of the SiC ingot 10 is referred to as an inner boundary 6, and a boundary located on an outer side from the inner boundary 6 in the XZ cut surface of the SiC ingot 10 is referred to as an outer boundary 7. In the SiC ingot 10 of the present embodiment shown in FIG. 1, the inner boundary 6 and the outer boundary 7 are straight lines inclined in the -X direction with respect to the Z direction (the crystal growth direction indicated by reference sign 8 in FIG. 1).

As shown in FIG. 1, the inner boundary 6 and the outer boundary 7 move toward the center of the SiC ingot 10 (-X direction: [-1-120] direction) as they are close to the second end 2 from the first end 1. That is, as the inner boundary 6 and the outer boundary 7 are close to the second end 2 from the first end 1, the facet 4 is positioned on a further inward side of the SiC ingot 10. As a result, the plane coordinates of the facet 4 at the first end 1 are different from the plane coordinates of the facet 4 at the second end 2.

In the actual SiC ingot 10, only the plane coordinates of the facet 4 at the first end 1 or the second end 2 can be checked, and the plane coordinates of the facet 4 inside can only be estimated. The facet 4 is a region in which the amount of a dopant element such as nitrogen taken in is greater than that in the step-flow growth region 5, and thus the facet 4 has a low resistance value and a low light transmittance. For this reason, when the SiC ingot 10 is processed into a cylindrical shape and then is subjected to laser processing, it is necessary to set the laser output conditions for the facet 4 and the step-flow growth region 5 to be different from each other. For example, if the facet 4 is present at a position within the SiC ingot 10 which is estimated from the plane coordinates of the facet 4 at the second end 2 that there will be no facet 4, the laser output may be insufficient during the laser processing. That is, in a case in which the SiC ingot 10 is processed into a cylindrical shape and then is subjected to the laser processing, there is a high possibility that defects will occur.

In contrast, in the SiC ingot 10 of the present embodiment, in the XZ cut surface (a cut surface that passes through the center and is in the <11-20> direction), an angle θ1 between the inner boundary 6 between the facet 4 and the step-flow growth region 5 and the crystal growth direction 8 is 56° or less. For this reason, the positional deviation between the plane coordinates of the facet 4 at the first end 1 and the plane coordinates of the facet 4 at the second end 2 is sufficiently small. Therefore, in a case in which the SiC ingot 10 is processed into a cylindrical shape and is subjected to the laser processing, processing stability is improved. The angle θ1 between the inner boundary 6 and the crystal growth direction 8 is preferably 50° or less, more preferably 45° or less, even more preferably 40° or less, still even more preferably 35° or less, and further more preferably 30° or less. The smaller the angle θ1 between the inner boundary 6 and the crystal growth direction 8, the better the processing stability becomes in a case in which the SiC ingot processed into a cylindrical shape is subjected to the laser processing, which is preferable. In addition, the SiC ingot 10 in which the angle θ1 between the inner boundary 6 and the crystal growth direction 8 is 10° or more can be easily manufactured, and is therefore preferable.

In the inner boundary 6 extending from the first end 1 toward the second end 2, an angle inclined in the [-1-120] direction with respect to a thickness direction from the first end 1 toward the second end 2 is preferably more than 0° and 56° or less. In other words, it is preferable that the inner boundary 6 be inclined in the -X direction with respect to the Z direction (the crystal growth direction indicated by reference sign 8 in FIG. 1). This is because it can be estimated from the plane coordinates of the facet 4 at the second end 2 that the facet 4 is not present at a region in the SiC ingot 10 on an inner side from the plane coordinates of the facet 4 at the second end 2. Therefore, in a case in which the SiC ingot 10 is processed into a cylindrical shape and is subjected to the laser processing, processing stability is further improved. The angle of inclination of the inner boundary 6 in the [-1-120] direction with respect to the thickness direction from the first end 1 toward the second end 2 is preferably 50° or less, more preferably 45° or less, even more preferably 40° or less, still even more preferably 35° or less, and further more preferably 30° or less.

In the SiC ingot 10 of the present embodiment, the angle θ1 between the inner boundary 6 and the crystal growth direction 8 is constant (in other words, the inner boundary 6 in the XZ cut surface is a straight line). The angle Θ1 between the inner boundary 6 and the crystal growth direction 8 in the SiC ingot 10 may vary depending on the position in the Z direction. In this case, the average value of the angles θ1 between the inner boundary 6 and the crystal growth direction 8 is treated as the angle θ1 between the inner boundary 6 and the crystal growth direction 8. The angle Θ1 between the inner boundary 6 and the crystal growth direction 8 is the average value of the angles Θ1 between the inner boundary 6 and the crystal growth direction 8 measured at five different positions in the Z direction.

In the SiC ingot 10 of the present embodiment, as shown in FIG. 1, a distance between the inner boundary 6 and the outer boundary 7 in the XZ cut surface preferably increases as the inner boundary 6 and the outer boundary 7 are close to the second end 2 from the first end 1. In other words, it is preferable that the area of the facet 4 at the second end 2 be larger than the area of the facet 4 at the first end 1. This is because the occurrence of crystals of heterogeneous polymorphs can be suppressed, and the SiC single crystal can be grown more stably. In addition, this is because it can be estimated from the plane coordinates of the facet 4 at the second end 2 that the facet 4 is not present at a region in the SiC ingot 10 which is wider than the plane coordinates of the facet 4 at the second end 2.

### "Method for manufacturing SiC ingot"

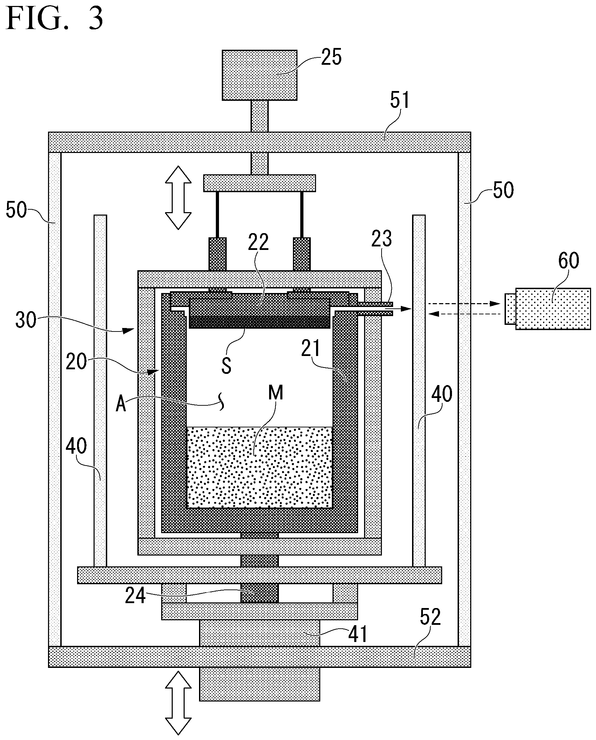

Next, a method for manufacturing the SiC ingot 10 according to the present embodiment will be described with reference to an example. FIG. 3 is a cross-sectional view showing an example of a manufacturing apparatus used when manufacturing the SiC ingot 10 according to the present embodiment. The apparatus for manufacturing the SiC ingot 10 shown in FIG. 3 includes a crucible 20, a heat insulating material 30, a liner quartz tube 40, a quartz tube 50, and a reflectance measuring instrument 60.

The crucible 20 is made of, for example, graphite. The crucible 20 includes an accommodation portion 21 and a lid 22. A gas discharge path 23 is formed between the accommodation portion 21 and the lid 22. The gas discharge path 23 is provided at least at one location on the outer side surface of the crucible 20 and may be provided at a plurality of locations. The gas discharge path 23 may be provided in a ring shape around the outer side surface of the crucible 20 between the accommodation portion 21 and the lid 22. The accommodation portion 21 is supported by and fixed to a support 24. The lid 22 is suspended by a suspension member 25 and can be moved up and down. A distance between the accommodation portion 21 and the lid 22 can be changed by moving the suspension member 25 up and down. That is, the width of the gas discharge path 23 in the Z direction can be freely designed.

A seed crystal S and a SiC raw material M are disposed in the film deposition space A in the crucible 20. Gas sublimated from the SiC raw material M is recrystallized on the surface of the seed crystal S, and thus the SiC single crystal that will become the crystal growth portion of the SiC ingot 10 is grown. A residual gas in the film deposition space A is discharged to the outside of the crucible 20 through the gas discharge path 23.

The atmospheric pressure in the film deposition space A during the crystal growth is set to be more than 0.3 Torr and less than 10 Torr. If the pressure in the film deposition space A is too low, a dopant element such as nitrogen cannot be sufficiently supplied into the crystal, and the resistivity of the SiC ingot 10 becomes high. The resistivity of the SiC ingot 10 is a parameter that affects the laser processing. In addition, if the pressure in the film deposition space A is too high, a sufficient amount of a sublimation gas is not generated from the SiC source material M; and as a result, productivity decreases.

The bulk density of the graphite forming the side surface of the crucible 20 is set to be more than 1.75 g/cm³ and less than 2.00 g/cm³. A part of the residual gas in the film deposition space A permeates through the crucible 20 and escapes to the outside. If the bulk density of the graphite forming the crucible 20 is low, a large amount of the residual gas will be discharged from portions other than the gas discharge path 23. The residual gas discharged from portions other than the gas discharge path 23 deteriorates the heat insulating material 30. On the other hand, if the bulk density of the graphite forming the crucible 20 is too high, the amount of thermal conductivity of the graphite becomes too large, and thus it is not possible to achieve an appropriate temperature distribution inside the crucible 20.

The volume G of the graphite forming the crucible 20 is set to be 1.5 to 4.0 times the volume F based on the diameter of the seed crystal S. The volume F corresponds to the inner volume of the crucible 20 and can be calculated by π × d³/2, where d is the diameter of the seed crystal S. If the volume G of the graphite forming the crucible 20 is small, the amount of the residual gas that permeates the crucible 20 increases, and this causes the heat insulating material 30 to deteriorate. If the volume G of the graphite is too large, the wall thickness of the crucible 20 becomes too large, and thus it is not possible to achieve an appropriate temperature distribution inside the crucible 20.

The heat insulating material 30 has an approximately cylindrical shape and is disposed to cover the periphery of the crucible 20. The volume of the heat insulating material 30 is more than one time and less than three times the volume of the crucible 20. The volume of the heat insulating material 30 is the volume of the heat insulating material 30 itself and is the volume of the area surrounded by the outer surface and the inner surface of the heat insulating material 30. The volume of the crucible 20 is the volume of the inside surrounded by the outer surface of the crucible 20. By setting the volume of the heat insulating material 30 within the above range, it is possible to achieve an appropriate temperature distribution inside the crucible 20. The temperature distribution inside the crucible 20 affects the formation of the facet 4.

The outer diameter of the heat insulating material 30 is set to be 1.2 times or more and 1.5 times or less the outer diameter of the crucible 20. The heat insulating material 30 located on the side surface of the crucible 20 is in direct contact with the crucible 20 which generates heat due to induction heating, and thus heat insulating performance is easily deteriorated. By setting the outer shape of the heat insulating material 30 within the above range, it is possible to achieve an appropriate temperature distribution inside the crucible 20 while preventing deterioration of the heat insulating material 30.

The liner quartz tube 40 is disposed around the heat insulating material 30. At least a part of the liner quartz tube 40 faces the end portion of the gas discharge path 23. The residual gas discharged from the gas discharge path 23 is blown onto the liner quartz tube 40, is adhered to the inner wall of the liner quartz tube 40, and is solidified. The reflectance of the liner quartz tube 40 increases due to the adhesion of the solidified residual gas. The liner quartz tube 40 is supported by a support 41. As the support 41 moves up and down, the liner quartz tube 40 also moves up and down. During the crystal growth, the liner quartz tube 40 is moved downward at a constant speed.

The quartz tube 50 surrounds the crucible 20, the heat insulating material 30, and the liner quartz tube 40. The quartz tube 50 is covered with an upper lid 51 and a lower lid 52. The quartz tube 50 makes it easier to control the atmosphere in the film deposition space A.

The reflectance measuring instrument 60 measures the reflectance of the portion of the liner quartz tube 40 to which the residual gas discharged from the gas discharge path 23 is applied. The reflectance of this portion increases as the amount of the gas discharged from the gas discharge path 23 increases. In the apparatus for manufacturing the SiC ingot 10 shown in FIG. 3, the amount of the gas discharged from the gas discharge path 23 is evaluated on the basis of the change in the reflectance of the liner quartz tube 40.

The liner quartz tube 40 is moved downward at a constant speed by the support 41 during the crystal growth. For this reason, the position in the liner quartz tube 40 to which the residual gas is applied is constantly changing. If the amount of the gas discharged from the gas discharge path 23 is constant, the reflectance of the portion of the liner quartz tube 40 of which the reflectance is measured by the reflectance measuring instrument 60 is constant.

In order to manufacture the SiC ingot 10 of which the angle Θ1 between the inner boundary 6 between the facet 4 and the step-flow growth region 5 and the crystal growth direction 8 is 56° or less, it is necessary to keep the flow of the sublimation gas in the film deposition space A approximately constant. When the amount of the gas discharged from the gas discharge path 23 changes, the flow of the sublimation gas in the film deposition space A changes.

In the apparatus for manufacturing the SiC ingot 10 shown in FIG. 3, in a case in which the reflectance of the liner quartz tube 40 which is measured by the reflectance measuring instrument 60 changes during the crystal growth, the width of the gas discharge path 23 can be changed by moving the suspension member 25 up and down. As a result, the amount of the gas discharged from the gas discharge path 23 is kept approximately constant.

For example, in a case in which the reflectance of the liner quartz tube 40 which is measured by the reflectance measuring instrument 60 decreases during the crystal growth, the amount of the gas discharged from the gas discharge path 23 is reduced, and thus the width of the gas discharge path 23 is to be increased. For example, in a case in which the reflectance of the liner quartz tube 40 which is measured by the reflectance measuring instrument 60 changes to be higher value during the crystal growth, the amount of the gas discharged from the gas discharge path 23 is increased, and thus the width of the gas discharge path 23 is to be reduced.

In the method for manufacturing the SiC ingot 10 of the present embodiment, as described above, the amount of the gas discharged from the gas discharge path 23 can be controlled by changing the width of the gas discharge path 23 in the manufacturing apparatus shown in FIG. 3. As a result, it is possible to control the temperature distribution and the flow of the sublimation gas within the film deposition space A, and it is possible to manufacture the SiC ingot 10 according to the present embodiment of which the angle θ1 between the inner boundary 6 between the facet 4 and the step-flow growth region 5 and the crystal growth direction 8 is 56° or less.

### (Another example)

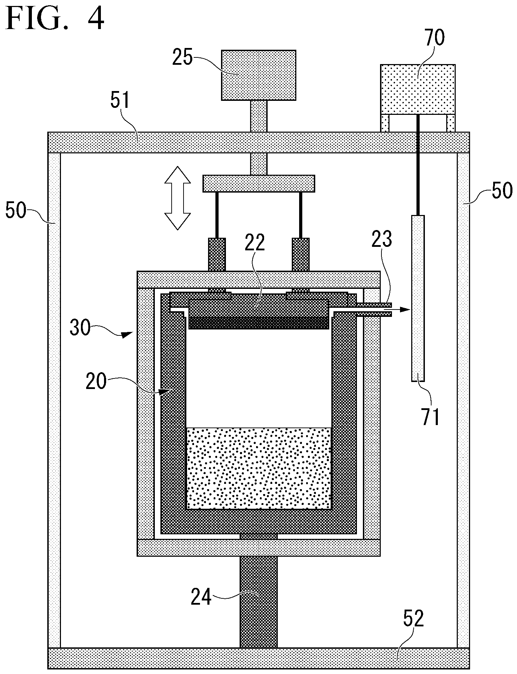

The SiC ingot 10 according to the present embodiment may be manufactured using a manufacturing method, which will be described below. FIG. 4 is a cross-sectional view showing another example of the manufacturing apparatus used when manufacturing the SiC ingot according to the present embodiment. The apparatus for manufacturing the SiC ingot 10 shown in FIG. 4 includes a crucible 20, a heat insulating material 30, a quartz tube 50, a weight measuring instrument 70, and an evaluation substrate 71.

The apparatus for manufacturing the SiC ingot 10 shown in FIG. 4 is provided with the evaluation substrate 71 instead of the liner quartz tube 40 in the apparatus for manufacturing the SiC ingot 10 shown in FIG. 3, and the weight measuring instrument 70 instead of the reflectance measuring instrument 60 in the apparatus for manufacturing the SiC ingot 10 shown in FIG. 3. The configurations of the crucible 20, the heat insulating material 30, and the quartz tube 50 in the apparatus for manufacturing the SiC ingot 10 shown in FIG. 4 are the same as those in the apparatus for manufacturing the SiC ingot 10 shown in FIG. 3.

In the apparatus for manufacturing the SiC ingot 10 shown in FIG. 4, the residual gas discharged from the gas discharge path 23 is blown onto the evaluation substrate 71. The residual gas blown onto the evaluation substrate 71 is adhered to the surface of the evaluation substrate 71 and is solidified. The weight of the evaluation substrate 71 increases due to the solidified residual gas.

The weight measuring instrument 70 measures the weight of the evaluation substrate 71. If the amount of the gas discharged from the gas discharge path 23 is constant, the acceleration of the weight increase of the evaluation substrate 71 is constant. In contrast, when the amount of the gas discharged from the gas discharge path 23 increases, the rate of the weight increase of the evaluation substrate 71 increases. In addition, when the amount of the gas discharged from the gas discharge path 23 decreases, the rate of the weight increase of the evaluation substrate 71 decreases.

In the apparatus for manufacturing the SiC ingot 10 shown in FIG. 4, the amount of the gas discharged from the gas discharge path 23 is evaluated on the basis of the change in the rate of the weight increase of the evaluation substrate 71.

In the method of manufacturing the SiC ingot 10 using the manufacturing apparatus shown in FIG. 4, as in the case of using the manufacturing apparatus shown in FIG. 3, the amount of the gas discharged from the gas discharge path 23 can be controlled by changing the width of the gas discharge path 23. As a result, it is possible to control the temperature distribution and the flow of the sublimation gas within the film deposition space A, and it is possible to manufacture the SiC ingot 10 according to the present embodiment of which the angle θ1 between the inner boundary 6 between the facet 4 and the step-flow growth region 5 and the crystal growth direction 8 is 56° or less.

In the above-described manufacturing method, as an example of a method for evaluating the amount of the gas discharged from the gas discharge path 23, the case in which a change in the reflectance of the liner quartz tube 40 or a change in the weight of the evaluation substrate 71 is used is described, but the physical quantity for evaluating the amount of the gas discharged from the gas discharge path 23 is not limited to the reflectance or the weight.

In the SiC ingot 10 of the present embodiment shown in FIG. 1, the shape of the facet 4 is controlled such that the positional deviation between the plane coordinates of the facet 4 at the first end 1 and the plane coordinates of the facet 4 at the second end 2 is sufficiently small. The SiC ingot 10 of the present embodiment can be processed by a known method to be a SiC ingot that is a cylindrical SiC single crystal.

In the SiC ingot processed into a cylindrical shape, as in the SiC ingot 10 of the present embodiment, in the XZ cut surface (a cut surface that passes through the center and is in the <11-20> direction), the angle θ1 between the inner boundary 6 between the facet 4 and the step-flow growth region 5 and the crystal growth direction 8 is 56° or less. For this reason, in the SiC ingot obtained by processing the SiC ingot 10 of the present embodiment into a cylindrical shape, the positional deviation between the plane coordinates of the facet 4 at the first end 1 and the plane coordinates of the facet 4 at the second end 2 is sufficiently small, and thus it is possible to reduce the number of times that the laser output is changed when cutting out the SiC substrate, and it is possible to easily and efficiently cut out the SiC substrate. The SiC ingot may have an orientation flat or a notch for identifying the direction of a crystal axis.

### "Method for manufacturing SiC substrate"

A method for manufacturing a SiC substrate of a first embodiment includes a step of manufacturing the SiC ingot 10 of the embodiment described above by any of the methods described above, a step of processing the SiC ingot 10 into a cylindrical shape, and a step of slicing the SiC ingot that has been processed into a cylindrical shape.

A known method can be used as the step for processing the SiC ingot 10 into a cylindrical shape. In addition, as the step of slicing the SiC ingot, for example, a method in which the SiC ingot is processed with a laser to create cracks and cut out the SiC substrates can be used.

In the present embodiment, in each of a plurality of SiC substrates cut out from the SiC ingot, as in the SiC ingot 10 of the present embodiment, in the XZ cut surface (a cut surface that passes through the center and is in the <11-20> direction), the angle θ1 between the inner boundary 6 between the facet 4 and the step-flow growth region 5 and the crystal growth direction 8 is 56° or less. In addition, in a case in which the angle θ1 between the inner boundary 6 and the Z direction (the crystal growth direction) of all of the plurality of SiC substrates cut out from the same SiC ingot is 56° or less, the SiC ingot before cutting out can be said to correspond to the SiC ingot of the present embodiment.

A method for manufacturing a SiC substrate of a second embodiment includes a step of preparing the SiC ingot 10 of the embodiment described above, and a step of slicing the SiC ingot 10.

The step of preparing the SiC ingot 10 may include obtaining the SiC ingot 10 of the above-mentioned embodiment from other companies, and the SiC ingot 10 may be a boule, provided that the boule meets the features of the SiC ingot 10 of the above-mentioned embodiment.

The step of slicing the SiC ingot 10 is the same as that in the first embodiment. The SiC ingot 10 may be processed into a cylindrical shape before the step of slicing the SiC ingot 10.

### "Method for evaluating SiC ingot"

A method for evaluating the SiC ingot 10 of the present embodiment includes a step of manufacturing the SiC ingot 10 of the present embodiment described above, a step of processing the SiC ingot 10 into a cylindrical shape, a step of slicing the SiC ingot that has been processed into a cylindrical shape to obtain a plurality of SiC substrates for evaluation, a step of measuring a position of the inner boundary 6 between the facet 4 and the step-flow growth region 5 in each SiC substrate for evaluation, a step of calculating the angle θ1 between the inner boundary 6 between the facet 4 and the step-flow growth region 5 in a cut surface that passes through a center of the SiC ingot 10 and is in a <11-20> direction and the crystal growth direction 8 on the basis of a relationship between a position in the SiC ingot from which each of the SiC substrates for evaluation is cut out and a position of the inner boundary 6 of each of the SiC substrates for evaluation, and an evaluation step.

In the evaluation step, it is evaluated whether the SiC ingot 10 is produced under conditions suitable for manufacturing a SiC ingot to be sliced by laser processing on the basis of the angle θ1 between the inner boundary 6 and the crystal growth direction 8 described above of the SiC ingot 10.

Specifically, the evaluation step is preferably a step of evaluating that the SiC ingot 10 is produced under conditions suitable for manufacturing a SiC ingot to be sliced by laser processing in a case in which the angle θ1 between the inner boundary 6 and the crystal growth direction 8 is 56° or less, and evaluating that the SiC ingot 10 is produced under conditions unsuitable for manufacturing a SiC ingot to be sliced by laser processing in a case in which the angle θ1 between the inner boundary 6 and the crystal growth direction 8 is more than 56°.

In addition, in the method for evaluating the SiC ingot 10 of the present embodiment, the position of the inner boundary 6 is measured for each of the plurality of SiC substrates for evaluation prepared in advance, and the results are used to calculate the angle θ1 between the inner boundary 6 and the crystal growth direction 8, and thus the shape of the facet in another SiC ingot manufactured by the same manufacturing method as the SiC ingot 10 may be evaluated. For this reason, according to the method for evaluating the SiC ingot 10 of the present embodiment, it is possible to evaluate with high accuracy whether the facet in another SiC ingot has a controlled shape suitable for a SiC ingot to be sliced by laser processing.

As described above, the preferable embodiments of the present invention have been described in detail, the present invention is not limited to specific embodiments, and various modifications and changes can be made within the scope of the features of the present invention described in the claims.

### [Examples]

### "Example 1"

Using the apparatus for manufacturing a SiC ingot shown in FIG. 3, two SiC ingots having a diameter of 160 mm and a thickness of 32.6 mm were produced while controlling the temperature distribution and the flow of the sublimation gas in the film deposition space A by adjusting the amount of the gas discharged from the gas discharge path 23.

The SiC ingots of Example 1 were visually seen in a plan view in the Z direction (the crystal growth direction). As a result, each of the SiC ingots of Example 1 included the step-flow growth region and the facet. In addition, one of the two SiC ingots of Example 1 was cut in the <11-20> direction passing through the center, and the inner boundary 6 between the facet 4 and the step-flow growth region 5 in the XZ cut surface was visually checked, and the angle θ1 between the inner boundary 6 and the Z direction (the crystal growth direction) was measured. As a result, θ1 was 27°.

In addition, the other SiC ingot 10 of Example 1 was processed into a cylindrical shape to obtain a SiC ingot having a diameter of 150 mm. The obtained SiC ingot was subjected to laser processing to create cracks, and 75 SiC substrates having a thickness of 0.35 mm were cut out.

The laser processing was performed under conditions in which the scan pitch was set to 200 µm, the feed speed of the laser scan was set to 200 mm/sec, the number of scans was set to 1, the acceleration/deceleration time was set to 0.1 sec, and the inter-line movement time was set to 0.1 sec. The acceleration/deceleration time was the time required to accelerate and decelerate when scanning with the laser in one direction and then scanning with the laser in an opposite direction. The inter-line movement time indicated the time required for movement in the X direction in a treatment in which laser scanning in the Y direction and the movement in the X direction were repeated.

By the above-described method, the time required to cut out 75 SiC substrates from the SiC ingot of Example 1 was 778 minutes. Therefore, the time required to cut out one SiC substrate was 10.4 minutes.

### "Example 2"

Two SiC ingots were produced in the same manner as in Example 1, except that the amount of the gas discharged from the gas discharge path 23 was adjusted to a value different from that in Example 1.

The SiC ingots of Example 2 were visually seen in a plan view in the Z direction (the crystal growth direction). As a result, each of the SiC ingots of Example 2 included the step-flow growth region and the facet. In addition, one of the two SiC ingots of Example 2 was cut in the <11-20> direction passing through the center, and the inner boundary 6 between the facet 4 and the step-flow growth region 5 in the XZ cut surface was visually checked, and the angle θ1 between the inner boundary 6 and the Z direction (the crystal growth direction) was measured. As a result, θ1 was 45°.

In addition, the other SiC ingot of Example 2 was processed into a cylindrical shape in the same manner as the SiC ingot of Example 1 to obtain a SiC ingot with a diameter of 150 mm. The obtained SiC ingot was subjected to laser processing in the same manner as in Example 1 to create cracks, and 75 SiC substrates having a thickness of 0.35 mm were cut out.

By the above-described method, the time required to cut out 75 SiC substrates from the SiC ingot of Example 2 was 833 minutes. Therefore, the time required to cut out one SiC substrate was 11.1 minutes.

### "Comparative Example 1"

Using a crucible that was the same as that used in Example 1 except that the gas discharge path 23 was not formed, the crystal growth of a SiC ingot was performed in a sealed crucible to produce two SiC ingots having a diameter of 159 mm and a thickness of 28 mm.

The SiC ingots of Comparative Example 1 were visually seen in a plan view in the Z direction (the crystal growth direction). As a result, each of the SiC ingots of Comparative Example 1 included the step-flow growth region and the facet. In addition, one of the two SiC ingots of Comparative Example 1 was cut in the <11-20> direction passing through the center, and the inner boundary 6 between the facet 4 and the step-flow growth region 5 in the XZ cut surface was visually checked, and the angle Θ1 between the inner boundary 6 and the Z direction (the crystal growth direction) was measured. As a result, θ1 was 62°.

In addition, the other SiC ingot of Comparative Example 1 was processed into a cylindrical shape in the same manner as the SiC ingot 10 of Example 1 to obtain a SiC ingot with a diameter of 150 mm. The obtained SiC ingot was subjected to laser processing in the same manner as in Example 1 to create cracks, and 64 SiC substrates having a thickness of 0.35 mm were cut out.

By the above-described method, the time required to cut out 64 SiC substrates from the SiC ingot of Comparative Example 1 was 761 minutes. Therefore, the time required to cut out one SiC substrate was 11.9 minutes.

The time required to cut out one SiC substrate from each of the SiC ingots of Examples 1 and 2 was shorter than the time required to cut out one SiC substrate from the SiC ingot of Comparative Example 1. The reason for this is that, in the case of the SiC ingots of Examples 1 and 2, the number of times that the laser output was changed when cutting out the SiC substrate from the SiC ingot was smaller than in the case of the SiC ingot of Comparative Example 1.

In Examples 1 and 2 and Comparative Example 1, the results for the SiC ingots with a diameter of 150 mm after processing have been shown so far. SiC ingots with a diameter of 200 mm after processing were also produced in the same manner, and a similar study was conducted for the SiC ingots with a diameter of 200 mm after processing. The same trend was observed in the case of 200 mm diameter as in the case of 150 mm diameter.

That is, the angle Θ1 between the inner boundary 6 and the Z direction (the crystal growth direction) in the XZ cut surface was 56° or less in the SiC ingot produced under the conditions of the embodiments. In contrast, the angle θ1 was more than 56° in the SiC ingot produced under the conditions that were out of the conditions of the embodiments. In addition, the time required to cut out one SiC substrate from the SiC ingot produced under the conditions of the embodiments was shorter than the time required to cut out one SiC substrate from the SiC ingot produced under the conditions that were out of the conditions of the embodiments.

### EXPLANATION OF REFERENCES

1 First end
2 Second end
3 Side wall
4 Facet
5 Step-flow growth region
10 SiC ingot
20 Crucible
21 Accommodation portion
22 Lid
23 Gas discharge path
24 Support
25 Suspension member
30 Heat insulating material
40 Liner quartz tube
41 Support
50 Quartz tube
51 Upper lid
52 Lower lid
60 Reflectance measuring instrument
70 Weight measuring instrument
71 Evaluation substrate

## Claims

1. A SiC ingot that is formed of a SiC single crystal grown from a first end inclined by an offset angle from a (0001) plane toward a second end, and
the SiC ingot comprising a step-flow growth region and a facet,
wherein, in a cut surface that passes through a center and is in a <11-20> direction, an angle between an inner boundary between the facet and the step-flow growth region and a crystal growth direction is 56° or less.

2. The SiC ingot according to claim 1, wherein, in the inner boundary extending from the first end toward the second end, an angle inclined in a [-1-120] direction with respect to a thickness direction from the first end toward the second end is more than 0° and 56° or less.

3. The SiC ingot according to claim 1 or 2, wherein a diameter is 149 mm or more.

4. The SiC ingot according to claim 1 or 2, wherein a diameter is 199 mm or more.

5. The SiC ingot according to any one of claims 1 to 4, wherein a maximum thickness between the first end and the second end in a direction perpendicular to the first end is 10 mm or more.

6. The SiC ingot according to any one of claims 1 to 4, wherein a maximum thickness between the first end and the second end in a direction perpendicular to the first end is 20 mm or more.

7. The SiC ingot according to any one of claims 1 to 4, wherein a maximum thickness between the first end and the second end in a direction perpendicular to the first end is 30 mm or more.

8. The SiC ingot according to any one of claims 1 to 4, wherein a maximum thickness between the first end and the second end in a direction perpendicular to the first end is 40 mm or more.

9. The SiC ingot according to any one of claims 1 to 4, wherein a maximum thickness between the first end and the second end in a direction perpendicular to the first end is 50 mm or more.

10. A method for manufacturing a SiC substrate comprising:
a step of manufacturing the SiC ingot according to any one of claims 1 to 9;
a step of processing the SiC ingot into a cylindrical shape; and
a step of slicing the SiC ingot that has been processed into a cylindrical shape.

11. A method for manufacturing a SiC substrate comprising:
a step of preparing the SiC ingot according to any one of claims 1 to 9; and
a step of slicing the SiC ingot.

12. A method for evaluating a SiC ingot comprising:
a step of manufacturing a SiC ingot;
a step of processing the SiC ingot into a cylindrical shape;
a step of slicing the SiC ingot that has been processed into a cylindrical shape to obtain a plurality of SiC substrates for evaluation;
a step of measuring a position of an inner boundary between a facet and a step-flow growth region in each SiC substrate for evaluation;
a step of calculating an angle between the inner boundary between the facet and the step-flow growth region in a cut surface that passes through a center of the SiC ingot and is in a <11-20> direction and a crystal growth direction on the basis of a relationship between a position in the SiC ingot from which each of the SiC substrates for evaluation is cut out and a position of the inner boundary of each of the SiC substrates for evaluation; and
an evaluation step of evaluating whether the SiC ingot is produced under conditions suitable for manufacturing a SiC ingot to be sliced by laser processing on the basis of the angle between the inner boundary and the crystal growth direction.
